Europäisches Patentamt

European Patent Office

Office européen des brevets

(11) Publication number: **0 195 881**
**A1**

(12) **EUROPEAN PATENT APPLICATION**

(21) Application number: **85850110.9**

(51) Int. Cl.⁴: **A 61 F 2/16**

(22) Date of filing: **27.03.85**

(43) Date of publication of application: **01.10.86**
**Bulletin 86/40**

(84) Designated Contracting States: **AT BE CH DE FR GB IT LI LU NL SE**

(71) Applicant: **PHARMACIA AB, Rapsgatan 7, S-751 82 Uppsala (SE)**

(72) Inventor: **Anker, Willem, Pinksterbloemweg 38, NL-9753 HG Haren (NL)**
Inventor: **Norrby, Sverker, De Grouw 29, NL-9351 LL Leek (NL)**

(74) Representative: **Svanfeldt, Hans-Ake et al, DR. LUDWIG BRANN PATENTBYRA AB Box 1344 Drottninggatan 7, S-751 43 Uppsala (SE)**

(54) **Intraocular lens.**

(57) The invention relates to intraocular lenses of the type suitable for implanting in the anterior chamber of a human eye. The lens comprises a lens body of for example poly-methyl-methacrylate (PMMA), two position fixation means extending tangentially outwards from opposite sides of the lens body, and gripping means on the position fixation means. A characteristic feature of the intraocular lens in accordance with the invention is that the position fixation means have a rectangular or semi-elliptical cross-section, whereby the lens is given an increased stiffness in a direction perpendicular to the plane of the lens, thus preventing it from vaulting.

1

# INTRAOCULAR LENS

The present invention relates to intraocular lenses of the type suitable for implanting in a human eye, as an artificial lens, and in particular to lenses of the type suitable for use in the anterior chamber of the eye.

For different reasons the lens of a human eye may become opaque. In severe cases the lens has to be removed. The eye sight of the patient can still be maintained by using special glasses with a very high power of the lens. This type of glasses however results in a very limited field of vision. Another method of maintaining the eye sight of the patient is to implant artificial lenses, referred to as intraocular lenses. These lenses are available in mainly three different types, anterior chamber lenses, iris-fixated lenses and posterior chamber lenses.

The anterior chamber lens is placed in the anterior chamber of the eye and is fixated in the anterior chamber angle.

A posterior chamber lens is fixated either in the capsular bag or the siliary sulcus following an extracapsular cataract extraction.

An iris-fixated lens is fixated to the iris eiher by sutures and/or clips.

A great number of anterior chamber lenses is known and available on the market. These lenses essentially comprise a light focusing lens body, made of some polymeric material, preferably poly-methyl-methacrylate (PMMA). The known lenses further comprise position fixation means, for seating in the interior of the eye and supporting the lens body. (Compare European Patent Applications 83303414.3, 83303415.0, 84300593.5, 84303881.1, European Patent Application Publication No. 0106488 and U.S. Patents Serial Nos. 4 298 995, 4 418 431, 4 370 760, and 4 174 543).

Intraocular lenses of this type according to prior art, suffer however from some disadvantages. Thus if an anterior chamber lens is too flexible in its characteristics, the lens can vault. For example when a person carrying a lens of this type, rubs his eye or if the eye is subjected to other external forces, the lens tends to vault.

The position fixation means of intraocular lenses according to prior art, have a wide variety of configurations. Thus the lens is supported at one or two points in the anterior chamber angle, or it is supported by a continous portion of the position fixation means. Supporting at one or two points only may not give a desired stability to the lens in the eye. Supporting the lens body with a continous portion of the position fixation means, tends on the other hand to irritate the tissue and even cause damage to the tissue in the eye.

Another drawback with intraocular lenses according to prior art, is that position fixation means having one or two supporting points inevitably implies that the actual insertion of the lens into the eye has to be carried out by slightly tilting the lens sideways. This tilting movement in the very narrow space available in the interior of the eye, may cause damage to the delicate tissue therein.

The object of the present invention is thus to provide an intraocular lens for insertion into the human eye, which overcomes the drawbacks mentioned above.

This object is achieved with the intraocular lens according to the attached patent claims.

Thus the intraocular lens according to the invention, comprises a light focusing body, first and second position fixation means, each comprising an inner and an outer part, said means being individually joined to and integral with the lens body and extending outwardly therefrom for seating in the anterior chamber angle, wherein at least the inner part of the position fixation means has a generally rectangular cross-section, such that the ratio between the major axis of the rectangle and the minor axis of the same rectangle is greater than 1.2 and smaller than 10, preferably 1.3-3 and that the major axis of the rectangle is perpendicular to the plane of the lens body. The rectangular cross-section of the position fixation means will increase the stiffness in a plane perpendicular to the lens body, thus preventing undesired vaulting of the lens body in the direction perpendicular to the plane of the lens body, and instead external forces are carried to create a rotational movement of the lens body in the plane of the lens.

After cutting the work piece of PMMA to the desired shape, e.g. with laser technique, the edges of the lens are bevelled sa as to give smoth contact to the eye tissue. This bevelling is achieved by e.g. tumbling.

In one embodiment of the invention the intraocular lens has diametrically opposite cut-in portions in the lens body adjacent the position fixation means, where these merge into the lens body. These cut-in portions in combination with the feature that the position fixation means merge tangentially into the generally circular lens body, will reduce the momentum of torque of the lens body thus facilitating its rotation in the plane of its body, thereby further preventing the lens body from vaulting.

The extent of this cut-in portion or insicion can vary, e.g. in that the width of the space between the lens body and the tangentially projecting position fixation means varies or in that the "length" of the insicion along the periphery of the lens body varies.

In another embodiment of the invention, the intraocular lens has on at least one of its position fixation means, gripping means, provided on the outer part of the position fixation means, at a point that coincides with the longitudinal central axis of the lens. This particular configuration of the gripping means, enables the surgeon performing the implantation of the lens, to insert the lens keeping it in generally one and the same plane throughout the operation of insertion. This is a great advantage compared to previously known intraocular lenses. With prior art lenses the surgeon whould have to tilt the lens slightly sideways during the insertion, with a great risk for damaging the delicate tissue of the interior of the eye. This risk is thus practically eliminated with the intraocular lens according to the present invention.

In still another embodiment of the invention, the lens is provided on the outer part of the position fixation means with a plurality of projections acting as supports for the lens in the anterior chamber angle. This plurality of projections gives a beneficial distribution of forces acting on the tissue, such that possible irritation experienced by a person carrying the lens (somtimes referred to as "tenderness-to-touch"), is significantly reduced.

Other features and advantages of the present invention is evident from the attached patent claims.

The invention will be described in detail below with reference to the attached drawings, in which

Fig. 1 is a top view of an intraocular lens according to the invention,

Fig. 2 is a partly sectional, partly side view of a lens in accordance with the invention along the longitudinal axis,

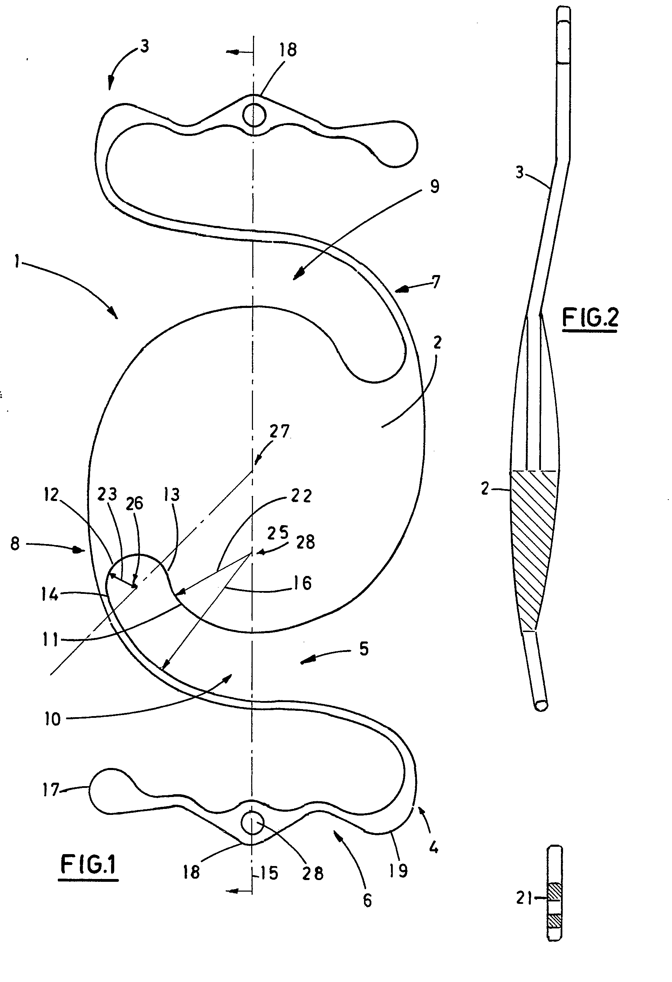

As shown in Fig. 1 of the drawings, an intraocular lens generally indicated at 1 comprises a light focusing lens body 2. The lens body may be constructed of any biologically inert and transparent material, preferably of PMMA. The lens includes first and second position fixation means or legs 3, 4, each comprising an inner and an outer part 5, 6. Since the diametrically opposed position fixation means are identical, reference will be made to only one throughout the description. These position fixation means are individually joined to and integral with the lens body and extend outwardly therefrom for seating in the anterior chamber angle of the eye. At least the inner part 5 of the legs 3 of the lens according to the invention have a generally rectangular cross-section. The major axis of this rectangular cross-section extends in a direction perpendicular to the plane of the lens body 2. The edges of the position fixation means 3, 4 can be bevelled, so as to make the cross-section semi-elliptical. This generally rectangular cross-section of the position fixation means 3, 4 is essential to the invention, in that it will impose an increased stiffness of the entire lens in a plane perpendicular to the lens body. This increased stiffnes thus prevents the lens body from vaulting outwards, i.e. in the direction perpendicular to the plane of the lens body. Instead any applied external forces, will be carried by the legs 3, 4 so as to create a rotational movement in the plane of the lens body.

As can be seen from Fig. 1 the intraocular lens of the invention has a lens body 2 with a generally circular form, known per se, from which lens body the legs 3, 4 project at two diametrically opposite points 7, 8 thereof. The legs project from the lens body in a direction generally tangentially to the periphery of the lens body.

Adjacent each said point 7, 8 there is a curved cut-in portion 9, 10 in the lens body 2. This cut-in portion comprises a first and a second curved portion 11, 12, said first curved portion starting tangential to the periphery of the lens body and continuing along an inwardly directed path to an end section 13. This end section is located at a first distance inwardly of the periphery of the lens body. The second curved portion 12 starts tangential to said end section 13 and continuous along an outwardly and backwardly directed path to an end section 14 located at a second distance outwardly of the periphery of the lens body and extending tangentially to the periphery of the lens body.

The path along which the first curved portion 11 extends can be described by an imaginary first circle, the centre of which is on the longitudinal axis 15 of the lens body and the radius 22 of which is smaller than that of the lens. The said first curved portion 11 extends along the said imaginary first circle through an angle of about 75°. The above mentioned second curved portion 12 extends along a second portion of a second imaginary circle the centre of which is falling on a line forming an angle of 45° which the longitudinal axis 15 of the lens and passing through the centre 27 of the lens body. The second curved portion 12 extends from the end section 13 of the first curved portion 11 along the imaginary second circle through an angle of about 180°. The radius 23 of said imaginary second circle is generally half of that of the first circle.

Each of the position fixation means of the lens is in the form of a curved leg, each leg starting from the end section 14 of the second curved portion 12 and running in a direction tangentially outwards from the lens body through an angle of about 75° along a third imaginary circle which has its centre 28 at that of the first imaginary circle and has a thrid radius 16 which is generally the sum of the radius of the first circle and the diameter of the second circle. The said portions form an inner part 5 of the leg running generally parallel to the periphery of the lens body and merging into said end section 14 of said second curved portion 12 in a direction extending tangentially to the periphery of the lens body.

In the embodiment of the invention according to Fig. 1 the ratio of the radius of the lens body and that of the first imaginary circle is in the order of about 2:1. The ratio between the radius of the first imaginary circle and that of the second imaginary circle is in the order of about 2,5:1. Finally the ratio of the radius of the lens body and that of the imaginary third circle is in the order of about 1:1 and the centre of the first circle divides the radius of the lens body in a proportion of 1:1.

As can be seen from Fig. 1 there is provided an outer part 6 of each leg 3, 4, said outer part being in the form of an upper portion of a S-shaped figure, the top of which is provided with a plurality of projections 17, 18, 19. One projection 18 is so loacted relative a corresponding one projection on the other leg that they both will fall on the longitudinal axis 15 of the lens. At least one of said projections 18 located on the longitudinal axis 15 is provided with a gripping means for gripping the lens during its implantation. This gripping means can be in the form of a hole 21 extending through the projection.

Each of the projections 17, 18, 19 on the position fixation means 3, 4 has a surface adapted to seat in the anterior chamber angle of the eye. Furthermore these surfaces are spaced along an imaginary circle corresponding to the contour line of the anterior chamber angle of the eye.

Referring now more particularly to Fig. 2, there is shown that the position fixation means are directed slightly out of the plane of the lens body. This is to secure that there is created a space between the lens body and the iris of the eye such that mechanical abrasion on the tissue is prevented or at least reduced.

The embodiment of the invention described herein is not intended to limit the invention. Thus the essense of the invention is that an intraocular lens is provided with position fixation means at least the inner part of which has a generally rectangular cross-section, cut-in portions adjacent the position fixation means, and gripping means for facilitating the insertion of the lens into the eye. These features are clearly indicated in the attached patent claims.

CLAIMS

1.  An intraocular lens (1) suitable for use as an artificial lens in the interior of a human eye, comprising a light focusing lens body (2) first and second position fixation means (3, 4), each comprising an inner and an outer part (5, 6), said means being individually joined to and integral with the lens body (2) and extending outwardly therefrom for seating in the anterior chamber angle, wherein at least the inner part (5) of the position fixation means has a generally rectangular cross-section, whereby the major axis of the rectangle extends in a direction perpendicular to the plane of the lens body (2), and whereby the ratio between the major and minor axes of said rectangule is greater than1,2 and smaller than 10.

2.  Intraocular lens in accordance with claim 1, wherein said ratio is preferably 1.3-3.

3.  An intraocular lens in accordance with claim 1, wherein the position fixation means are bevelled so as to have a semi-elliptical cross-section.

4.  An intraocular lens in accordance with claim 3, wherein the lens body (2) has a generally circular form, known per se and where the position fixation means (3, 4), in a manner known per se, project from the lens body at two diametrically opposite points (7, 8) thereof, wherein said position fixation means are projecting from the lens body (2) in a direction generally tangentially to the circular lens body.

5.  An intraocular lens in accordance with claim 4, wherein the lens body (2) has a cut-in portion, bordered by an outer curved surface (11) of the lens body (2), said curved surface having a curvature that is smaller than the curvature of the periphery of the lens body, further bordered by the surface extending along a semi-circle the center of which lies on the principal circular arc of the lens, and also bordered by the inner surface of the inner part (5) of the position fixation means (3, 4).

6.   An intraocular lens in accordance with claim 5, wherein each said position fixation means (3, 4) is in the form of a curved leg, and each said leg, starting from the end section of a second curved portion (12), runs in a direction tangentially outward from the lens body (2) through an angle of about 75° along an imaginary circle, which has its centre (28) at the centre (25) of another  imaginary circle, and has a radius (16) which is generally the sum of the radius (22) of the latter circle and the diameter of the second curved portion (12), thereby to form an inner part (5) of the leg, running generally parallel to the periphery of the lens body and merging into said end section (14) of said second curved portion (12) in a direction extending tangentially to the periphery of the lens body.

7.   An intraocular lens in accordance with claim 6, wherein the outer part (6) of each leg is in the form of an upper portion of a S-shaped figure, the top of which is provided with a plurality of projections (17, 18, 19), in a wave-shaped configuration, one projection (18) on one leg being so located relative a corresponding one projection (18) on the other leg, that they both will fall on the longitudinal axis (15) of the lens, whereby at least one of said projections (18) is provided with a gripping means for gripping the lens during its implantation.

8.   An intraocular lens in accordance with claim 7, wherein said gripping means is in the form of a hole (21) extending through the projection (18).

9. An intraocular lens in accordance with claim 8, wherein said projections (17, 18, 19), each has a surface adapted to seat in the anterior chamber angle of the eye, said surfaces being spaced along an imaginary circle corresponding to the coutour line of the anterior chamber angle of the eye.

10. An intraocular lens suitable for use as an artificial lens in the interior of a human eye, comprising a light focussing lens body (2), first and second position fixation means (3, 4), each comprising an inner and an outer part (5, 6), said means being individually joined to and integral with the lens body (2) and extending outwardly therefrom for seating in the anterior chamber angle, wherein the position-fixation means (3, 4) are in the form of legs, at least the inner part (5) of which has a generally rectangular cross-section, the major axis of which extends in the direction perpendicular to the plane of the lens body (2), and whereby the ratio between the major and minor axes of said rectangle is greater than 1.2 and smaller than 10 the outer part (6) of each leg being in the form of an upper portion of a S-shaped figure, the top of which is provided with a plurality of projections (17, 18, 19), one projection (18) on one leg being so located relative a corresponding one projection (18) of the other leg that they both will fall on the longitudinal axis (15) of the lens, whereby at least one of said projections (18) is provided with a gripping means for gripping the lens during its implantation.

11. Intraocular lens in accordance with claim 10, wherein the ratio is preferably 1.3-3.

12. An intraocular lens in accordance with claim 10, wherein the position fixation means (3, 4) are bevelled so as to have a semi-elliptical cross-section.

12

13. An intraocular lens in accordance with claim 10, wherein said gripping means is in the form of a hole (21) extending through the projection (18).

14. An intraocular lens in accordance with claim 10, wherein each of said projections (17, 18, 19), has a surface adapted to seat in the anterior chamber angle of the eye, said surfaces being spaced along an imaginary circle corresponding to the contour line of the anterior chamber angle of the eye.

---

1/1

0195881

FIG.1

FIG.2

European Patent
Office

**EUROPEAN SEARCH REPORT**

Application number

EP 85 85 0110

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 100 989 (J.L. TENNANT) * Page 2, lines 66-81; page 3, lines 4-19; figures 1-10 * | 1-4 | A 61 F 2/16 |
| Y | | 5-14 | |
| | --- | | |
| Y | US-A-4 504 981 (G.B. WALMAN) * Column 7, lines 1,2,26-42; column 9, lines 30-38; figure 3 * | 5,6 | |
| | --- | | |
| Y | US-A-4 249 271 (S. POLER) * Figure 12 * | 7-14 | |
| | --- | | |
| A | US-A-4 437 194 (G.L. HAHS) * Column 5, lines 8-19,52-54; figure 2 * | 1,2 | |
| | --- | | TECHNICAL FIELDS SEARCHED (Int. Cl.4) |
| A | US-A-4 502 162 (G.J. GERHARD et al.) * Column 3, line 50 - column 4, line 2; figures 1,3 * | 1-4 | A 61 F |
| | --- | | |
| A | US-A-4 343 050 (C.D. KELMAN) * Figures 4,6 * | 7,10 | |
| | --- | | |
| D,A | GB-A-2 111 835 (F.T. FEASTER) * Abstract; page 3, lines 44-52; figure 9 * | 4,5,7, 10 | |
| | ---     -/- | | |

The present search report has been drawn up for all claims

| Place of search THE HAGUE | Date of completion of the search 22-11-1985 | Examiner WOLF C.H.S. |
|---|---|---|

European Patent Office
**EUROPEAN SEARCH REPORT**
Application number

| | **DOCUMENTS CONSIDERED TO BE RELEVANT** | | | Page 2 |
|---|---|---|---|---|
| Category | Citation of document with indication, where appropriate, of relevant passages | Relevant to claim | | CLASSIFICATION OF THE APPLICATION (Int. Cl.4) |
| D,A | EP-A-0 106 488 (E.J. ARNOTT) <br> * Page 3, lines 3-30; page 5, lines 10-14; page 7, line 26 - page 8, line 11; figure 1 * | 4-6 | | |
| D,A | EP-A-0 130 710 (T.C. WHITE) <br> * Page 8, lines 1-11, line 31 - page 9, line 6; page 13, line 9 - page 14, line 21; figures 3,7,9 * <br> ----- | 4-10 | | |
| | | | | **TECHNICAL FIELDS SEARCHED (Int. Cl.4)** |
| | The present search report has been drawn up for all claims | | | |

| Place of search <br> THE HAGUE | Date of completion of the search <br> 22-11-1985 | Examiner <br> WOLF C.H.S. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82